Europäisches Patentamt

European Patent Office

Office européen des brevets

(19)

(11) **EP 0 410 430 B1**

(12) **EUROPÄISCHE PATENTSCHRIFT**

(45) Veröffentlichungstag und Bekanntmachung des Hinweises auf die Patenterteilung:
03.01.1996 Patentblatt 1996/01

(51) Int Cl.⁶: **C12P 13/00**

(21) Anmeldenummer: **90114287.7**

(22) Anmeldetag: **25.07.1990**

(54) **Verfahren zur diskontinuierlichen Herstellung von L-Carnitin auf mikrobiologischem Weg**

Process for the microbiological discontinual preparation of L-carnitine

Procédé de préparation microbiologique discontinue de L-carnitine

(84) Benannte Vertragsstaaten:
**AT BE CH DE DK ES FR GB IT LI LU NL SE**

(30) Priorität: **28.07.1989 CH 2813/89**

(43) Veröffentlichungstag der Anmeldung:
**30.01.1991 Patentblatt 1991/05**

(73) Patentinhaber: **LONZA AG**
**CH-3945 Gampel/Wallis (CH)**

(72) Erfinder: **Hoeks, Frans, Dipl.-Ing.**
**Naters (Kanton Wallis) (CH)**

(74) Vertreter: **Weinhold, Peter, Dr. et al**
**D-80803 München (DE)**

(56) Entgegenhaltungen:
**EP-A- 0 122 794       EP-A- 0 195 944**

**Beschreibung**

Die Erfindung betrifft ein neues Verfahren zur Herstellung von L-Carnitin auf mikrobiologischem Weg.

L-Carnitin ist eine für den menschlichen Metabolismus essentielle Substanz für z.B. den Abbau der Fettsäuren. Künstlich hergestelltes L-Carnitin wird daher in pharmazeutischen Präparaten als Wirksubstanz gegen entsprechende Mangelkrankheiten eingesetzt.

Es ist bekannt, L-Carnitin aus $\gamma$-Butyrobetain herzustellen. Das $\gamma$-Butyrobetain wird in Gegenwart von Natrium-2-oxoglutarat, einem reduzierenden Agens, einer Eisenionenquelle und Luftsauerstoff als Hydroxylgruppendonator mit einem Hydroxylase-Enzym, freigesetzt aus Sporen von Neurospora crassa, in Berührung gebracht (US-PS 4 371 618). Dieses Verfahren weist den Nachteil auf, eine Vielzahl von Cofaktoren zu benötigen, die extern zugeführt werden müssen. So werden in der Reaktion stöchiometrische Mengen 2-Oxoglutarat oxidativ zu Succinat decarboxyliert. $Fe^{2+}$ wird als $O_2$-Aktivator benötigt, Ascorbat, um das Eisen-Ion in der reduzierten Form zu halten, und Katalase, um das in Spuren entstehende, schädliche $H_2O_2$ zu zerstören.

Lindstedt et al, [Biochemistry 6, 1262-1270 (1967) "The Formation and Degradation of Carnitin in Pseudomonas"] isolierten einen Mikroorganismus der Gattung Pseudomonas, der mit $\gamma$-Butyrobetain als C- und N-Quelle wächst Die erste Reaktion des Abbauweges war die Hydroxylierung des $\gamma$-Butyrobetains zu L-Carnitin, wobei das intermediär entstehende L-Carnitin vollständig zu $CO_2$, $H_2O$ und $NH_3$ weiterkatabolisiert wurde.

Auch diese, aus Bakterien gewonnene Hydroxylase hätte, wenn sie für die L-Carnitin-Produktion eingesetzt würde, die geschilderten nachteiligen Cofaktor-Bedürfnisse [Lindstedt et al, Biochemistry 16, 2181- 2188 (1977) "Purification and Properties of $\gamma$-Butyrobetaine Hydroxylase from Pseudomonas sp. AK 1"].

Gemäss US-PS 4 708 936 ist im weiteren bekannt, L-Carnitin auf mikrobiologischem Weg kontinuierlich herzustellen. Es hat sich aber als nachteilig herausgestellt, dass die Stammstabilität beim kontinuierlichen Verfahren sehr hoch sein muss (mehr als 1000 h), ausserdem sind der Produktkonzentration im Medium relativ niedrige Grenzen gesetzt. Das Produkt/Eduktverhältnis ist ebenfalls relativ niedrig.

Die EP-A-0 122 794 offenbart ein mikrobiologisches Verfahren zur L-Carnitin-Herstellung ausgehend von Crotonobetain, das als klassischer Batch-Prozeß durchgeführt wird. In diesem Verfahren wird Crotonobetain als Edukt mit Hilfe von Mikroorganismen, beispielsweise der Gattungen Escherichia oder Pseudomonas, die Crotonobetain verstoffwechseln können, zu L-Carnitin transformiert, wobei das gesamte Ausgangsmaterial zusammen mit einer Auswahl von Nährstoffen und Spurenelementen bereits zu Beginn des Verfahrens im Kulturmedium vorliegt. Die hierbei im Kulturmedium erreichbaren L-Carnitin-Konzentrationen liegen unter 1%, so daß dieses Verfahren für eine Produktion in technischem Maßstab nicht zufriedenstellend ist.

Die Aufgabe der vorliegenden Erfindung ist es, die Nachteile der bekannten Verfahren zu vermeiden und ein Verfahren zu finden, das es gestattet, enantioselektiv und auf mikrobiologischem Weg in hoher Ausbeute L-Carnitin aus Crotonobetain und oder $\gamma$-Butyrobetain herzustellen.

Diese Aufgabe konnte durch ein Verfahren zur diskontinuierlichen Herstellung von L-Carnitin durch mikrobiologische Verwertung von Crotonobetain und/oder $\gamma$-Butyrobetain gelöst werden, das dadurch gekennzeichnet ist, daß einem Kulturmedium enthaltend einen Mikroorganismus der Gattung Pseudomonas, Rhizobium, Agrobacterium, E. coli oder einer Hefe der Gattung Saccharomyces das Crotonobetain und/oder $\gamma$-Butyrobetain, Betain und eine zusätzliche Kohlenstoffquelle kontinuierlich zudosiert werden und nach Erreichen der maximalen L-Carnitin-Konzentration das L-Carnitin abgetrennt wird.

Zweckmässig werden Mikroorganismen der Gattung Rhizobium, vorzugsweise der Stamm HK 1331b, hinterlegt am 8.2.1985 bei der Deutschen Sammlung von Mikroorganismen (DSM), Gesellschaft für Biotechnologische Forschung mbH, Griesebachstr. 8, D-3400 Göttingen, unter der Nr. DSM 3225, der Stamm HK 13, hinterlegt am 23.1.1984 bei derselben Stelle, unter der DSM Nr. 2903 verwendet.

Für das Verfahren ebenso geeignet, sind Mikroorganismusstämme, die gentechnologisch verändert sind.

Im Unterschied zu den im Stand der Technik bekannten Systemen verwenden die erfindungsgemässen Mikroorganismen das $H_2O$, und nicht das $O_2$, als Hydroxylgruppendonator, wie durch eigene Untersuchungen unter Verwendung von $H_2{}^{18}O$ und $^{18}O_2$ festgestellt werden konnte.

Die Selektion und die Charakterisierung dieser bevorzugten Mikroorganismen kann nach der in der EP-A 0 158 194 beschriebenen Selektionsmethode erfolgen:

a) Mikroorganismen, die mit Betain, $\gamma$-Butyrobetain, Crotonobetain und L-Carnitin als C- und N-Quelle wachsen, werden auf übliche Weise mutiert.

b) Aus der durch Züchtung erhaltenen Kultur von mutierten Mikroorganismen werden jene selektioniert, die stabil sind, L-Carnitin nicht katabolisieren und nicht auf L-Carnitin, Crotonobetain, $\gamma$-Butyrobetain wachsen, wohl aber mit Betain.

Vorzugsweise werden folgend auf Selektionsschritt b) jene Mikroorganismen ausgewählt, die L-Carnitin ausscheiden und nicht auf L-Carnitin, Crotonobetain, γ-Butyrobetain wachsen, wohl aber mit Betain.

Zweckmässig werden die mutierten Mikroorganismen in einem Betain-Medium weiterkultiviert und diese weiterkultivierten Mikroorganismen zur Durchführung des Selektionsschrittes b) vorzugsweise in einem L-Carnitin-Medium weitergezüchtet. Die Züchtung von mit Betain, γ-Butyrobetain, Crotonobetain und L-Carnitin als C- und N-Quelle wachsenden Stämme wird zweckmässig so durchgeführt, dass man aus Bakteriengemischen durch Beimpfung von Crotonobetain-Nährlösungen Mischkulturen erzeugt und aus denen unter Zuhilfenahme traditioneller mikrobiologischer Techniken dann Reinkulturen von Crotonobetain abbauenden Mikroorganismen anlegt.

Die Mutation einer solchen Kultur, die mit Betain, γ-Butyrobetain, Crotonobetain und L-Carnitin als C- und N-Quelle wächst, kann nach bekannten Methoden durchgeführt werden [J.H. Miller, Experiments in Molecular Genetics, Cold Spring Harbor Laboratory, 1972].

Zweckmässig anwendbare Methoden zur Herstellung stabiler Mutanten sind die Frame-Shift-Methode, Deletionsmethode oder die Transposon-Insertionsmethode. Die so mutierten Mikroorganismen werden dann nach der Weiterkultivierung in einem Betain-Medium und der Ueberführung in ein L-Carnitin-Medium dem Selektionsschritt b) unterzogen, wo mittels bekannten "counter-selektionierenden Agenzien" [P.Gerhardt et al (eds.), Manual of Methods for General Bacteriology, Am.Soc. for Microbiology, 1981] jene Mikroorganismen selektioniert werden, die stabil L-Carnitin nicht katabolisieren und nicht auf L-Carnitin, Crotonobetain, γ-Butyrobetain wachsen, wohl aber mit Betain.

Das erfindungsgemässe Verfahren zur Herstellung von L-Carnitin wird zweckmässig derart ausgeführt, dass man in einer ersten sogenannten Batch-Phase produktionsfähige Biomasse erzeugt. Dazu wird auf bekannte Weise entsprechend der EP-A 0 158 194 einer der genannten Stämme in einem sterilisierten, vorzugsweise vitaminhaltigen Mineralmedium [Kulla et al, Arch. Microbiol. 135, 1 (1983)] bei 20 bis 40°C, vorzugsweise bei 30°C, bei einem zweckmässigen pH-Wert von 6 bis 8, vorzugsweise 7, während 5 bis 80 h, vorteilhaft während 15 bis 40 h, kultiviert. Dieses Medium enthält zweckmässig 0,01 bis 10 Gew.%, vorzugsweise 0,01 bis 5 Gew.%, Cholin, Glutamat, Acetat, Dimethylglycin oder Betain als Wachstumssubstrat. Besonders bevorzugt wird Betain zusammen mit Glutamat oder einer weiteren Kohlenstoffquelle in Mengen von je 0,02 bis 5 Gew.% eingesetzt.

Des weiteren wird in der Batch-Phase die umzusetzenden Ausgangsverbindungen γ-Butyrobetain, Crotonobetain oder Gemische davon in Mengen von 0,01 bis 10 Gew.%, vorzugsweise 0,1 bis 5 Gew.%, bezogen auf das Reaktionsmedium, vorgelegt. Das γ-Butyrobetain bzw. Crotonobetain kann teilweise als Hydrochloridsalz oder als freies inneres Salz vorliegen.

Mit der in der Batch-Phase gezüchteten Biomasse können weitere Kulturen beimpft werden. Diese weiteren Kulturen haben zweckmässig die gleiche Zusammensetzung wie die Vorkulturen.

Beim erfindungsgemässen Verfahren ist die in der Batch-Phase gezüchtete Biomasse Ausgangspunkt für die L-Carnitinproduktion in der sog. "fed-batch"-Phase.

Das Kulturmedium in der "fed-batch"-Phase ist dem der Batch-Phase weitgehend ähnlich.

Die "fed-batch"-Phase ist dadurch charakterisiert, dass der Kulturlösung das Edukt Crotonobetain und/oder γ-Butyrobetain, Betain sowie eine zusätzliche Kohlenstoffquelle zudosiert wird.

Als Kohlenstoffquelle können die in der Fachwelt üblichen und z.B. für Rhizobiumstämme literaturbekannten Verbindungen eingesetzt werden [The prokaryotes, Chapter 67, The genus Rhizobium, Springer Verlag 1981, p. 825].

Zweckmässige Kohlenstoffquellen sind z.B. Zucker wie Glucose oder Fructose, Zuckeralkohole wie Glycerin oder org. Säuren wie Essigsäure.

Vorzugsweise wird Glucose oder Glycerin eingesetzt.

Das Molverhältnis Kohlenstoff zu Stickstoff (C zu N) wird zweckmässig zwischen grösser 5 Mol C zu 1 Mol N und 10'000 Mol C zu 1 Mol N, vorzugsweise zwischen 10 Mol C zu 1 Mol N und 100 Mol C zu 1 Mol N, gewählt.

Bei sehr hohen Kohlenstoff-Stickstoff-Verhältnissen kann es notwendig sein, neben dem Betain eine weitere Stickstoffquelle zuzusetzen. Als weitere Stickstoffquelle können dazu die in der Fachwelt üblichen und bekannten Vertreter wie z.B. Ammonium verwendet werden. Es können auch zusätzliche Nährstoffe, wie z.B. eine Schwefel-, eine Phosphorquelle, Spurenelemente, Vitamine oder komplexe Nährstoffe, wie Fleischextrakte, Hefeextrakte oder Maisquellwasser, zudosiert werden.

Die Edukte γ-Butyrobetain oder Crotonobetain werden zweckmässig so zugeführt, dass im Kulturmedium eine Konzentration zwischen zweckmässig 0,005 bis 5%, vorzugsweise zwischen 0,02 und 2%, gewährleistet ist.

Die Zugabegeschwindigkeit der Kohlenstoff/Stickstoffquelle ist abhängig von der Biomassemenge im Bioreaktor. Eine bekannte Methode zur Bestimmung der Biomassekonzentration ist die Messung des Trockengewichts der Kulturlösung. Diesem Trockengewicht entsprechend und im Hinblick auf das gewünschte Kohlenstoff/Stickstoffverhältnis wird die Kohlenstoffquelle und Betain mit einer Geschwindigkeit von zweckmässig 0,01 bis 100, vorzugsweise von 0,1 bis 10 Mol Kohlenstoff pro kg Trockengewicht im Fermenter pro Stunde dem Fermenter zudosiert.

Es wird vorteilhaft in einem Temperaturbereich von 20 bis 40°C bei einem pH zwischen 6 und 8 gearbeitet.

Nach Kultivierung in der "fed-batch"-Phase von in der Regel nach 25 bis 250 h kann eine L-Carnitin-Konzentration in der Kultur von mehr als 6% erreicht werden.

Nach einer Modifikation des Verfahrens ist es auch möglich, nach Beendigung der Fermentation einen Teil der Kulturlösung abzulassen und mit dem verbleibenden Teil, aufgefüllt mit neuem Kulturmedium, eine erneute "fed-batch"-Kultivierung anzufahren.

Mit diesem sog. "repeated fed-batch"-Verfahren gelingt es, die volumetrische Produktivität der Fermentation zu erhöhen.

Eine Entlastung der Kultur kann dadurch erreicht werden, dass man das einzusetzende γ-Butyrobetain und/oder das Crotonobetain vorgängig mittels Ionenaustauscher oder mittels Elektrodialyse entsalzt und reinigt.

Die Gewinnung des L-Carnitins aus der Kulturlösung ist z.B. aus der EP-A 195 944 bekannt und kann so durchgeführt werden, dass nach Abtrennung der Biomasse durch z.B. Zentrifugieren, Ultrafiltration oder Mikrofiltration mittels einer Labor-Elektrodialyseanlage die Lösung von den geladenen Teilchen (Kationen und Anionen) befreit wird. Der Endpunkt der Entsalzung kann konduktometrisch ermittelt werden. Dabei wandern die Salze in den Konzentratkreislauf, während das L-Carnitin als inneres Salz ("Betain") im Diluat-Kreislauf verbleibt. So können Ausbeuten an L-Carnitin im Diluat nach der Entsalzung von mehr als 95% erreicht werden.

Alternativ zur Elektrodialyse kann das L-Carnitin auch mittels eines stark sauren Kationenaustauschers in der $H^{\oplus}$-Form entsalzt werden [vgl. J.P. Vandecasteele, Appl. Environ. Microbiol. 39, 327 (1980)]. Dabei lässt man die Lösung solange über eine Ionenaustauscherkolonne fliessen, bis der Ionenaustauscher erschöpft ist und L-Carnitin durchbricht. Die Anionen gehen als freie Säuren in den Durchlauf. Die Kationen bleiben auf dem Ionenaustauscher. Nach Neutralwaschen des Ionenaustauschers mit Wasser kann das L-Carnitin mit wässriger Ammoniaklösung eluiert werden. So können Ausbeuten an L-Carnitin im ammoniakalischen Eluat von mehr als 95% erreicht werden.

Die sowohl bei der Elektrodialyse wie auch mittels Ionenaustauscher anfallenden verdünnten L-Carnitin-Lösungen können entweder durch Verdampfung oder auch durch Umkehrosmose aufkonzentriert und anschliessend azeotrop entwässert werden.

Das so anfallende L-Carnitin kann dann durch anschliessende Umkristallisation aus zweckmässig Isobutanol/Aceton, Methanol, Ethanol, n-Butanol, bzw. in Kombination mit Lösungsmitteln, welche L-Carnitin wenig lösen, wie z.B. Aceton, Ethylacetat, γ-Butylacetat, Isobutylmethylketon und Acetonitril, vorzugsweise Isobutanol, und zusätzliche Aktivkohlebehandlung in reines weisses L-Carnitin übergeführt werden. Es kann gemäss diesem Verfahren L-Carnitin mit spezifischen Drehungen von $[\alpha]_D^{25}$ -30,5 bis -31,0°, c = 1 in $H_2O$ [Literaturwert -30,9°; Strack et al, Hoppe-Seyler's Z.f.physiolog.Chem., 318 (1960), 129] und einem Gehalt von mehr als 99% (HPLC) erhalten werden.

Beispiel 1

Eine 0,3 l Vorkultur des Stammes HK 1331b wurde in folgendem Nährmedium bei 30°C, pH 7,0 während 24 h kultiviert:

Zusammensetzung des Nährmediums

| L-Glutamat | 2 g |
|---|---|
| Betain | 2 g |
| γ-Butyrobetain | 2 g |
| Puffer-Lösung | 100 ml |
| Mg-Ca-Fe-Lösung | 25 ml |
| Spurenelementen-Lösung | 1 ml |
| Vitamin-Lösung | 1 ml |
| mit Wasser auf | 1 l |

| Puffer-Lösung | |
|---|---|
| $Na_2SO_4$ | 1 g |
| $Na_2HPO_4 \cdot 2H_2O$ | 25,08 g |
| $KH_2PO_4$ | 10 g |
| NaCl | 30 g |
| mit Wasser auf | 1 l |

| Mg-Ca-Fe-Lösung | |
|---|---|
| $MgCl_2 \cdot 6H_2O$ | 16 g |
| $CaCl_2 \cdot 2H_2O$ | 0,58 g |
| $FeCl_3 \cdot 6H_2O$ | 0,032 g |
| mit Wasser auf | 1 l |

| Spurenelementen-Lösung | |
|---|---|
| $ZnSO_4 \cdot 7H_2O$ | 100 mg |
| $MnCl_2 \cdot 4H_2O$ | 30 mg |
| $H_3BO_3$ | 300 mg |
| $CoCl_2 \cdot 6H_2O$ | 200 mg |
| $CuCl_2 \cdot 2H_2O$ | 10 mg |
| $NiCl_2 \cdot 6H_2O$ | 22 mg |
| $Na_2MoO_4 \cdot 2H_2O$ | 30 mg |
| mit Wasser auf | 1 l |

| Vitamin-Lösung | |
|---|---|
| Pyridoxal.HCl | 10 mg |
| Riboflavin | 5 mg |
| Nikotinamid | 5 mg |
| Thiamin.HCl | 5 mg |
| Biotin | 2 mg |
| Natriumpantothenat | 5 mg |
| p-Aminobenzoesäure | 5 mg |
| Folsäure | 2 mg |
| Vitamin B 12 | 5 mg |
| mit Wasser auf | 1 l |

Mit der Vorkultur wurden 5 l Nährmedium gleicher Zusammensetzung im Fermenter beimpft und bei 30°C, pH 7, während 24 h kultiviert. Der pH wurde durch Zugabe von 8%iger Phosphorsäure bei 7,0 konstant gehalten.

a) Anschliessend wurde mit dem "fed-batch" Betrieb begonnen. Zwei Lösungen folgender Zusammensetzungen wurden kontinuierlich zudosiert.

| Zusammensetzung des Kohlenstoff-Stickstoffzulaufs | |
|---|---|
| Betain | 100 g |
| Glucose | 135 g |
| mit Wasser auf | 1 l |
| Kohlenstoff/Stickstoffverhältnis | 10,3:1 |

| Zusammensetzung des γ-Butyrobetainzulaufs | |
|---|---|
| γ-Butyrobetain | 300 g |
| mit Wasser auf | 1 l |

Die Lösung mit der Kohlenstoff- und der Stickstoffquelle wurde mit einer Geschwindigkeit von 4,5 ml/h zudosiert. Das entsprach einer spezifischen Zulaufgeschwindigkeit von 4 Mol C/kg Trockengewicht/h am Anfang der "fed-batch"-Phase. Das Trockengewicht wurde auf übliche Weise bestimmt (z.B. Appl. Microbiol. Biotechnol 28 [1988] 109f.). Die Konzentration an γ-Butyrobetain und L-Carnitin wurde mittels HPLC bestimmt. Die γ-Butyrobetainlösung wurde derart zudosiert, dass die γ-Butyrobetainkonzentration im Fermenter zwischen 0,05 und 0,5 Gew.% lag. 150 h nach der Inokulierung des Fermenters wurde eine L-Carnitinkonzentration von 6,4% und eine Konzentration von nicht umgesetztem γ-Butyrobetain von 0,29% erreicht. Dies entsprach einem 95%igen Umsatz von γ-Butyrobetain.

b) Entsprechend a) und mit der selben Zusammensetzung des Kohlenstoff/Stickstoffzulaufs und des γ-Butyrobetainzulaufs wurde der "fed-batch"-Betrieb aufgenommen.

Die Lösung mit der Kohlenstoff- und der Stickstoffquelle wurde mit einer Geschwindigkeit von 4,5 ml/h zudosiert. Das entsprach einer spezifischen Zulaufgeschwindigkeit von 4 Mol C/kg Trockengewicht/h am Anfang der "fed-batch"-Phase. Das Trockengewicht wurde auf übliche Weise bestimmt (z.B. Appl. Microbiol. Biotechnol 28 [1988] 109f.). Die Konzentration an γ-Butyrobetain und L-Carnitin wurde mittels HPLC bestimmt. Die γ-Butyrobetainlösung wurde derart zudosiert, dass die γ-Butyrobetainkonzentration im Fermenter zwischen 0,05 und 0,15 Gew.% lag. 155 h nach der Inokulierung des Fermenters wurde eine L-Carnitinkonzentration von 6,3% und eine Konzentration von nicht umgesetztem γ-Butyrobetain von 0,13% erreicht. Dies entsprach einem 98%igen Umsatz von γ-Butyrobetain.

## Isolierung von L-Carnitin

Reines L-Carnitin konnte aus der Lösung, welche 64 g/l L-Carnitin, 2,9 g/l γ-Butyrobetain und anorganische Salze enthielt, entsprechend dem Vorgehen aus der EP-A 195 944 isoliert werden. Nach der Reinigungsstufe durch Umkristallisation konnten 56,3 g (88%) weisses L-Carnitin, HPLC > 99%, spez. Drehung $\alpha_D^{25}$ -30,9°, (c=1, $H_2O$) erhalten werden.

## Beispiel 2

300 ml des im Beispiel 1 beschriebenen Nährmediums, das anstelle des γ-Butyrobetains 2 g/l Crotonobetain enthielt, wurde mit dem Stamme HK 1331b angeimpft und bei 30°C, pH 7, während 24 h kultiviert.

Mit der Vorkultur wurde 5 l Nährmedium wie in Beispiel 1 beimpft und bei 30°C, bei pH 7,0 während 24 h kultiviert. Durch Zugabe von 8%iger Phosphorsäure wurde der pH bei 7,0 konstant gehalten. Anschliessend wurde mit dem "fed-batch" Betrieb begonnen. Wie in Beispiel 1 beschrieben wurde die Kohlenstoff- und Stickstoffquelle sowie eine Lösung nachstehender Zusammensetzung kontinuierlich zudosiert.

| Zusammensetzung des Crotonobetainzulaufs | |
|---|---|
| Crotonobetain | 300 g |
| mit Wasser auf | 1 l |

Die Lösung mit der Kohlenstoff- und der Stickstoffquelle wurde mit einer Geschwindigkeit von 4,5 ml/h zudosiert. Das entsprach einer spezifischen Zulaufgeschwindigkeit von ca. 4 Mol C/kg Trockengewicht/h am Anfang der "fed-batch"-Phase. Die Muster wurden auf derselben Art und Weise, wie beschrieben im Beispiel 1, behandelt und analysiert. Der Crotonobetainzulauf wurde derart zudosiert, dass die Crotonobetainkonzentration im Fermenter zwischen 0,05 und 0,5 Gew.% lag. 150 h nach der Inokulierung des Fermenters wurde eine L-Carnitinkonzentration von 6,1% und eine Konzentration von nichtumgesetztem Crotonobetain von 0,17% erreicht. Dies entsprach einem 95%igen Umsatz von Crotonobetain.

## Isolierung von L-Carnitin

Reines L-Carnitin konnte aus der Lösung, welche 61 g/l L-Carnitin, 1,7 g/l Crotonobetain und anorganische Salze enthielt, entsprechend der EP-A 195 944 aufgearbeitet werden.

Nach der Umkristallisation konnten 52 g (86%) weisses L-Carnitin mit den gleichen Spezifikationen wie in Beispiel 1 erhalten werden.

## Patentansprüche

1. Verfahren zur diskontinuierlichen Herstellung von L-Carnitin durch mikrobiologische Verwertung von Crotonobetain und/oder γ-Butyrobetain, dadurch gekennzeichnet, daß einem Kulturmedium enthaltend einen Mikroorganismus der Gattung Pseudomonas, Rhizobium, Agrobacterium, E. coli oder einer Hefe der Gattung Saccharomyces das Crotonobetain und/oder γ-Butyrobetain, Betain und eine zusätzliche Kohlenstoffquelle kontinuierlich zudosiert werden und nach Erreichen der maximalen L-Carnitin-Konzentration das L-Carnitin abgetrennt wird.

2. Verfahren nach Anspruch 1, dadurch gekennzeichnet, daß das Crotonobetain und/oder γ-Butyrobetain so zudosiert wird, daß die Konzentration im Kulturmedium zwichen 0,005 und 5% liegt.

3. Verfahren nach irgendeinem der Ansprüche 1 und 2, dadurch gekennzeichnet, daß das Betain und die zusätzliche Kohlenstoffquelle so zudosiert werden, daß ein Kohlenstoff-Stickstoff Molverhältnis von 10.000 zu 1 bis größer 5 zu 1 eingehalten wird.

4. Verfahren nach irgendeinem der Ansprüche 1 bis 3, dadurch gekennzeichnet, daß als zusätzliche Kohlenstoffquelle Zucker, Zuckeralkohole oder organische Säuren eingesetzt werden.

5. Verfahren nach Anspruch 4, dadurch gekennzeichnet, daß als zusätzliche Kohlenstoffquelle Glucose oder Glycerin eingesetzt wird.

6. Verfahren nach irgendeinem der Ansprüche 1 bis 5, dadurch gekennzeichnet, daß die Zugabegeschwindigkeit von Betain und Kohlenstoffquelle zwischen 0,01 und 100 Mol Kohlenstoff pro kg Trockengewicht im Fermenter pro Stunde beträgt.


**Claims**

1. Process for the batchwise preparation of L-carnitine by microbiological utilization of crotonobetaine and/or γ-butyrobetaine, characterized in that the crotonobetaine and/or γ-butyrobetaine, betaine and a supplemental carbon source are continuously proportioned to a culture medium containing a microorganism of the genus Pseudomonas, Rhizobium, Agrobacterium, E. coli or a yeast of the genus Saccharomyces and the L-carnitine is isolated after the maximum concentration of L-carnitin has been reached.

2. Process according to claim 1, characterized in that the crotonobetaine and/or γ-butyrobetaine are proportioned so that the concentration in the culture medium is between 0.005 and 5%.

3. Process according to any one of claims 1 and 2, characterized in that the betaine and the supplemental carbon source are proportioned so that a molar ratio of carbon-nitrogen of from 10000 to 1 to more than 5 to 1 is maintained.

4. Process according to any one of claims 1 to 3, characterized in that as supplemental carbon source sugars, sugar alcohols or organic acids are utilized.

5. Process according to claim 4 characterized in that as supplemental carbon source glucose or glycerol are utilized.

6. Process according to any one of claims 1 to 5, characterized in that the supplementation rate of betaine and carbon source is between 0.01 and 100 moles of carbon per kg dry weight in the fermenter per hour.


**Revendications**

1. Procédé pour la préparation discontinue de L-carnitine par utilisation microbiologique de crotonbétaïne et/ou de γ-butyrobétaïne, caractérisé en ce que l'on ajoute de façon dosée à un milieu de culture contenant un microorganisme du typé pseudomonas, rhizobium, agrobacterium, E. coli ou à une levure de type saccharomyces, la crotonbétaïne et/ou la γ-butyrobétaïne, la bétaïne et une source de carbone supplémentaire de façon continue et après obtention de la concentration de L-carnitine maximum, on sépare la L-carnitine.

2. Procédé selon la revendication 1, caractérisé en ce que l'on ajoute de façon dosé la crotonbétaïne et/ou la γ-butyrobétaïne de façon que la concentration se situe entre 0,005 et 5% dans le milieu de culture.

3. Procédé selon l'une quelconque des revendications 1 et 2, caractérisé en ce que l'on ajoute de façon dosée la bétaïne et la source de carbone supplémentaire de façon à maintenir un rapport molaire entre le carbone et l'azote de 10.000 à 1 jusqu'à un rapport supérieur de 5 à 1.

4. Procédé selon l'une quelconque des revendications 1 à 3, caractérisé en ce qu'en tant que source de carbone supplémentaire, on utilise du sucre, des alcools de sucre ou des acides organiques.

5. Procédé selon la revendication 4, caractérisé en ce qu'en tant que source de carbone supplémentaire, on utilise du glucose ou de la glycérine.

6. Procédé selon l'une quelconque des revendications 1 à 5, caractérisé en ce que la vitesse d'addition de la bétaïne et de la source de carbone dans l'appareil de fermentation se situe entre 0,01 et 100 moles de carbone par kilo poids sec par heure.